(19) Europäisches Patentamt
European Patent Office
Office européen des brevets

(11) EP 1 034 801 A1

# (12) EUROPEAN PATENT APPLICATION

(43) Date of publication:
**13.09.2000 Bulletin 2000/37**

(51) Int. Cl.[7]: **A61L 15/46**

(21) Application number: **99103909.0**

(22) Date of filing: **05.03.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant:
**THE PROCTER & GAMBLE COMPANY**
**Cincinnati, Ohio 45202 (US)**

(72) Inventors:
- **Pesce, Antonella**
  **65120 Pescara (IT)**
- **Carlucci, Giovanni**
  **66100 Chieti (IT)**
- **Di Cintio, Achille**
  **65126 Pescara (IT)**
- **Gagliardini, Alessandro**
  **60035 Jesi (IT)**

(74) Representative:
**Kremer, Véronique Marie Joséphine et al**
**Procter & Gamble**
**European Service GmbH**
**65823 Schwalbach am Taunus (DE)**

## (54) Absorbent articles having an odour control system comprising an oxidising agent and a chelating agent

(57) The present invention relates to disposable absorbent articles for controlling odours, especially odours associated with bodily fluids, (preferably sanitary napkins and pantiliners) which comprise an oxidising agent, preferably a peroxyacid and/or diacyl peroxide, together with a chelating agent, as the odour control system. In a preferred embodiement the articles further comprise an absorbing gelling material and/or an additional odour control agent.



Printed by Xerox (UK) Business Services
2.16.7 (HRS)/3.6

## Description

Field of the Invention

This invention relates to absorbent articles for controlling odour, especially odour associated with bodily fluids, comprising an oxidising agent together with a chelating agent.

Background of the Invention

Malodours may be present in the environment from numerous sources both animate and inanimate. Many products and articles are available which aim to avoid or minimise the detection of such odours. In particular, it is particularly desirable to provide odour control materials to address the malodours which are generated by the human body, or from bodily fluids such as perspiration, urine, faeces, menstrual fluids, vaginal fluids and the like.

Articles like absorbent articles for example are designed to be worn by humans to absorb bodily fluids, such as urine, menstrual fluid and perspiration, etc. Examples of absorbent articles include sanitary napkins, pantiliners, disposable diapers, incontinence pads, tampons, perspiration pads, and the like.

In use, the absorbent articles are known to acquire a variety of compounds, for example volatile fatty acids (e.g. isovaleric acid), ammonia, amines (e.g. triethylamine), sulphur containing compounds (e.g. mercaptans, sulphides), alcohols, ketones and aldehydes (e.g., furaldehyde) which release unpleasant odours. These compounds may be present in the bodily fluid or may be developed by chemical reactions and/or any fluid degradation mechanisms once the bodily fluid is absorbed into the absorbent article like for example a feminine pad. In addition bodily fluids usually contain microorganisms and/or enzymes that can also generate malodorous by products as a result of degradation mechanisms like putrefactive degradation, acid degradation, proteins degradation, fat degradation and the like. Unpleasant odours which emanate from absorbent pads when in use may make the wearer feel self conscious.

Various odour control materials have been disclosed in the art to combat some of the unpleasant odours referred to above. Indeed solutions have been provided that use different technical approaches like masking, i.e., covering the odour with a perfume, or absorbing the odour already present in the bodily fluids and those generated after degradation.

Most of the focus in the prior art is found on the odour absorption technology. Examples of these types of compounds include activated carbons, clays, zeolites, silicates, starches, cyclodextrine, ion exchange resins and various mixture thereof as for example described in EP-A-348 978, EP-A-510 619, WO 91/12029, WO 91/11977, WO89/02698, and/or WO 91/12030. All of these types of odour absorbing agents are believed to control odour by mechanisms whereby the malodorous compounds and their precursors are physically absorbed by the agents and thus such agents hinder the exit of the odour from absorbent articles. However, such mechanisms are not completely effective as the formation of the odour itself is not prevented and thus odour detection is not completely avoided.

Thus although these materials provide some control of odours associated with bodily fluids, there still exists a need of further improvement in terms of odour control over a wider range of malodorous compounds.

It is an object of the present invention to provide effective odour control over a wider range of malodorous compounds. More particularly, it is an object of the present invention to provide disposable absorbent articles which deliver a broader spectrum of odour control.

It has now been found that the above needs can be addressed by combining a chelating agent together with an oxidising agent, as the odour control system for a disposable absorbent article.

It has been surprisingly discovered that the combination of a chelating agent, preferably ethylene diamine tetracetate (EDTA) together with an oxidising agent, preferably a peroxyacid like ε-phtalimido peroxyhexanoic acid (PAP), or a diacyl peroxide like dibenzoyl peroxide, benzoyl lauroyl peroxide and/or dilauroyl peroxide in an absorbent article coming into contact with bodily fluids, results in a synergistic effect in terms of odour control. Indeed this combination gives more odour reduction than the odour reduction associated to the use of one of these agents alone at the same total level (either the chelating agent alone or the oxidising agent alone) in an absorbent article coming into contact with bodily fluids.

Actually the combination of the oxidising agent with a chelating agent in an article herein allows to combine odour control mechanisms by which the malodor detection is synergistically reduced or even prevented as well as odor control over a very broad odour spectrum.

It is speculated that the oxidising agents herein have a dual odour control mechanism: they are able to prevent the generation of odour by blocking enzymatic and/or microbial activity as well as to combat the odours already present by oxidising them into no-smelling molecules.

It is further speculated that the chelating agents due to their exceptional ability to remove iron, copper, calcium, magnesium and manganese ions present in the absorbed fluids block the microbial metabolism by the formation of chelates, thereby preventing the formation of malodours.

It is further speculated that the presence of the chelating agent boosts the oxidising capacity of the oxidising agent. Indeed it is speculated that the presence of the chelating agent reduces the decomposition of the oxidising agent when it comes into contact with an environment enriched in metal ions like copper, iron, calcium, magnesium and manganese typically present in bodily fluids, which would otherwise have inactivated the oxidising agents. It is believed that the presence of the chelating agent allows to retain the full oxidising capacity of the oxidising agent up to the time/moment it comes into contact with the body fluid as well as effective oxidising capacity over prolonged periods of time once into contact with the bodily fluid. Hence, an advantage of the present invention is that the present combination contributes to use less oxidising agent in the odour control system whilst maintaining the required level of odour control.

A further advantage associated with the preferred absorbent articles of the present invention is that they deliver a better feeling and more acceptable cleanness level for the person wearing them. Indeed, the present invention due to the presence of oxidizing agents as described herein provides an absorbent article capable of changing the color of the menstruation (red blood color) to a pale red color and even to a whitish color.

In a preferred embodiment herein the absorbent articles herein have an apertured polymeric film topsheet. This topsheet contributes to a further improved odour control of the odour control system herein.

Whereas the present invention is preferably directed to absorbent articles like pantiliners, feminine napkins, incontinent pads, diapers, tampons, interlabial pads, perspiration pads, surgical pads, breast pads and the like, other articles may include the odour control system as described herein too for the purpose of effective odour control. Indeed, other applications include other absorbent articles designed to be worn in contact with the body such as bandages, thermal pads, acne pads, cold pads, compresses, surgical pads/dressings and the like, body and household cleansing articles like impregnated wipes (e.g. baby wipes, wipes for feminine intimate hygiene), impregnated tissues, towels, articles for absorbing perspiration such as shoe insoles, shirt inserts, carpets and the like, and articles for animals like litters and the like.

## Background art of the invention

US 4363322 discloses deodorising and disinfecting liquid-absorbing products such as a sanitary napkin, a compress or a diaper, comprising a liquid absorbing material and inside the product at a distance from its outer edges a substance which gives off oxygen in contact with moisture like peroxides, ozonides, superoxides, oxo-ozonides and the like. This reference does not disclose the presence of any chelating agent according to the present invention let alone the preferred ones like ethylene diamine tetracetate.

EP-A-811391 discloses absorbent articles comprising an apertured polymeric film topsheet, a backsheet and an absorbent core, said article comprising an odour control system comprising a chelating agent. However, EP-A-811391 fails to disclose oxidising agents, let alone the outstanding odour control benefit associated to the combination of oxidising agents like peroxyacids and/or diacyl peroxides, with chelating agents.

## Summary of the Invention

The present invention relates to a disposable absorbent article for controlling odour, preferably odour associated with bodily fluids, comprising at least one oxidising agent and at least one chelating agent. In a preferred embodiment of the invention the article also comprises an absorbing gelling material and/or an additional odour control agent.

## Detailed description of the Invention

The articles according to the present invention, for controlling odour, especially odour associated with bodily fluids, comprise as an essential element an oxidising agent and a chelating agent.

By "absorbent article" it is meant herein any tridimentional solid material being able to absorb fluids and to receive/carry an odour control system as described herein comprising at least one oxidising agent and at least one chelating agent. Suitable disposable absorbent articles according to the present invention are those designed to be worn in contact with the body of a user and to receive fluids discharged from the body, such as disposable absorbent pantiliners, sanitary napkins, catamenials, incontinence inserts/pads, diapers, tampons, breast pads, interlabial pads/inserts and the like. Other articles suitable according to the present invention also include other articles designed to be worn in contact with the body such as bandages, thermal pads, acne pads, cold pads, compresses, surgical pads/dressings and the like, articles for absorbing perspiration such as shoe insoles, shirt inserts, perspiration pads and the like, body and household cleansing articles like impregnated wipes (e.g. baby wipes, wipes for feminine intimate hygiene), impregnated tissues, towels, and the like, and articles for animals like litters and the like.

By "bodily fluids" it is meant herein any fluids produced by human or animal body occurring naturally or accidentally like for instance in the case of skin culling, including for instance perspiration, urine, menstrual fluids, faeces, vaginal secretions and the like.

The oxidising agent

The oxidising agent according to the present invention can be any oxidising agent known to those skilled in the art able to impact on malodorous compounds to reduce malodour associated therewith. They include oxygen bleaches.

Suitable oxygen bleaches for use herein include peroxygen bleaches or mixtures thereof.

Such peroxygen bleaches include hydrogen peroxide, percarbonates, persilicates, persulphates, perphosphates, peroxyacids, alkyl hydroperoxides, peroxides, diacyl peroxides, ozonides, supereoxides, oxo-ozonides, periodates, salts thereof or mixtures thereof. Peroxyacids and diacyl peroxides are preferred herein.

Suitable hydroperoxides for use herein include for instance tert-butyl hydroperoxide, cumyl hydroperoxide, 2,4,4-trimethylpentyl-2-hydroperoxide, di-isopropylbenzene-monohydroperoxide, tert-amyl hydroperoxide and 2,5-dimethyl-hexane-2,5-dihydroperoxide.

Suitable peroxides for use herein include for example lithium peroxide, sodium peroxide, potassium peroxide, ammonium peroxide, calcium peroxide, rubidium peroxide, cesium peroxide, strontium peroxide, barium peroxide, magnesium peroxide, mercury peroxide, silver peroxide, zirconium peroxide, hafnium peroxide, titanium peroxide, phosphorus peroxide, sulphur peroxide, rhenium peroxide, cobalt peroxide, nickel peroxide, other alkali metal salts thereof or alkaline earth metal salts thereof or mixture thereof.

Suitable superoxides for use herein include for example lithium superoxide, sodium superoxide, potassium superoxide, calcium superoxide, rubidium superoxide, cesium superoxide, stromtium superoxide, barium superoxide, other alkali metal salts thereof or alkaline earth metal salts thereof or mixture thereof.

Suitable ozonides for use herein include for example lithium ozonide, sodium ozonide, potassium ozonide, rubidium ozonide, cesium ozonide, ammonium ozonide, tetramethyl ammonium ozonide, stromtium ozonide, barium ozonide, magnesium ozonide, other alkali metal salts thereof or alkaline earth metal salts thereof or mixture thereof.

Suitable perphosphates for use herein include for example lithium perphosphate, sodium perphosphate, potassium perphosphate, zinc perphosphate, ammonium perphosphate, calcium perphosphate, barium perphosphate, magnesium perphosphate, other alkali metal salts thereof or alkaline earth metal salts thereof or mixture thereof.

Suitable persulphates for use herein include sodium persulphate, potassiumdipersulphate, potassium persulphate as well as other alkali metal salts thereof or mixture thereof.

Other suitable peroxygen bleaches also include dicetylperoxydicarbonate, 1,1 bis(terbutylperoxy)-3,3,5-trimetylcyclohexane, di(1-naphtyl) peroxide, tert-butyl perbenzoate, O,O,-t-buthyl-O-isopropyl mono-peroxycarbonate, percarbonates like stearyl percarbonate, 2-ethylhexyl percarbonate and sec-butyl percarbonate and corresponding persulphates.

Suitable diacyl peroxides for use herein are according to the formula:

$$R_1\text{-C(O)-O-O-(O)C-}R_2$$

wherein $R_1$ and $R_2$ can be the same or different and are selected from the group of substituted or unsubstituted, saturated or unsaturated, linear, branched or cyclic hydrocarbon groups having from 1 to 50 carbon atoms, preferably from 2 to 40 and more preferably from 4 to 30 carbon atoms.

Suitable diacyl peroxides for use herein include those wherein $R_1$ and $R_2$ are independently an aliphatic group, those wherein $R_1$ and $R_2$ are independently an aromatic ring and those wherein $R_1$ is an aliphatic group and $R_2$ is an aromatic ring.

Typically $R_1$ and $R_2$ are independently an aliphatic group having from 2 to 40, more preferably 4 to 30, even more preferably 5 to 20 carbon atoms. These aliphatic groups may be linear, branched, cyclic, saturated, unsaturated, substituted, unsubstituted or mixtures thereof Preferably the aliphatic groups are linear and comprise from 4 to 20 carbon atoms, and more preferably 8 to 18 carbon atoms. Where such an aliphatic group is substituted, the carbon atom is preferably substituted with a halide or sulfate-containing or nitrogen-containing functionality such as SO3-, SO4-, NO2, NR3+ where R = H or an alkyl chain containing from 1 to 5 carbon atoms.

Typically $R_1$ and $R_2$ may be independently a mono or polycyclic aromatic ring, a homo or heteroaromatic ring, substituted or unsubstituted having from 2 to 50 carbon atoms and mixtures thereof. Where such an aromatic ring is substituted, the carbon atom is preferably substituted with a halide, sulphur-containing group, nitrogen-containing group or an alkyl chain wherein the number of carbon atoms ranges from 1 to 20, most preferably from 4 to 10. Suitable sulphur-containing or nitrogen-containing substituents include SO3-, SO4-, NO2, NR3+ where R=H or an alkyl chain containing from 1 to 5 carbon atoms. Preferred aromatic ring is benzene.

Particularly suitable diacyl peroxides for use herein are those wherein $R_1$ is an aliphatic group as defined herein before and $R_2$ is a mono or polycyclic aromatic ring, a homo or heteroaromatic ring, substituted or unsubstituted as defined herein before. Such preferred diacyl peroxides are benzoyl alkanoyl peroxides wherein the alkanoyl group has from 4 to 20 carbon atoms and more preferably from 8 to 18 carbon atoms.

Suitable diacyl peroxides for use herein are dilauroyl peroxide, didecanoyl peroxide, dimyristoyl peroxide, dibenzoyl peroxide, di-4-methylbenzoyl peroxide, di-p-methoxy-benzoyl peroxide, acetyl benzoyl peroxide, benzoyl stearoyl peroxide, benzoyl decanoyl peroxide, benzoyl cetyl peroxide, para-alkyl benzoyl lauroyl peroxide, para-alkyl benzoyl decanoyl peroxide, para-alkyl benzoyl cetoyl peroxide, di-4-phenylbenzoyl peroxide, di-t-butylperoxide, t-butyl cumyl peroxide, diethyl peroxide, diacetyl peroxide, dicumyl peroxide, diheptanoyl peroxide, didecanoyl peroxide, benzoyl lauroyl peroxide, diheptanoyl peroxide, distearoyl peroxide, disuccinyl peroxide, 3,5,5-trimethylhexanoyl peroxide, or mixtures thereof.

Highly preferred diacyl peroxides herein are dilauroyl peroxide which may be commercially available as flakes from AKZO NOBEL under the name Laurox®, or as powder under the name Laurox S®, or in suspension in water under the name Laurox W 40®, or dibenzoyl peroxide which may be commercially available from AKZO NOBEL under the name Lucidol® in powder form or Lucidol W40® in the form of a suspension in water, and/or benzoyl lauroyl peroxide.

The aromatic alkanoyl peroxides described herein like benzoyl lauroyl peroxide are easily synthesized by persons skilled in the art, see for example Organic Peroxides Vol. 1; page 65, edited by Daniel Swern of Wiley Interscience.

Suitable peroxyacids for use herein are according to the following formulae:

$$R_3\text{-CO3H}$$

wherein $R_3$ is a substituted or unsubstituted, saturated or unsaturated, linear or branched hydrocarbon group having from 1 to 25 carbon atoms or a cyclic group having from 3 to 32 carbon atoms and optionally at least one heteroatom or a cyclic alkyl group having from 4 to 32 carbon atoms and optionally at least one heteroatom.

Typically $R_3$ is a substituted or unsubstituted, linear or branched alkyl group or alkenyl group having from 1 to 25 carbon atoms, more preferably from 1 to 14 carbon atoms, even more preferably from 3 to 10, and most preferably from 4 to 6. $R_3$ may also typically be an aryl group having from 3 to 32 carbon atoms, preferably from 3 to 25, more preferably from 6 to 20, even more preferably from 8 to 15 carbons atoms, or an aryl alkyl group having from 4 to 32 total carbon atoms, preferably from 4 to 25, more preferably from 6 to 20 and even more preferably from 8 to 13, or an heterocyclic group containing from 3 to 32 carbon atoms, preferably from 3 to 25, more preferably from 3 to 20 carbon atoms, even more preferably from 5 to 15 and from 1 to 5 hetero atoms, preferably 1 to 3, wherein the hetero atoms are independently selected from the group consisting of oxygen, nitrogen and sulfur, and preferably is nitrogen or oxygen, or an heterocyclic alkyl group containing from 4 to 32 total carbon atoms, preferably from 4 to 25, more preferably from 4 to 22, even more preferably from 6 to 18 and from 1 to 5 hetero atoms, preferably 1 to 3, wherein the hetero atoms are independently selected from the group consisting of oxygen, nitrogen and sulfur, and preferably is nitrogen or oxygen.

The preferred peroxyacids according to the present invention are those wherein $R_3$ is a cyclic group or cyclic alkyl group, preferably a heterocyclic group or heterocyclic alkyl group.

Even more preferred herein are the peroxyacids according to the following formulae:

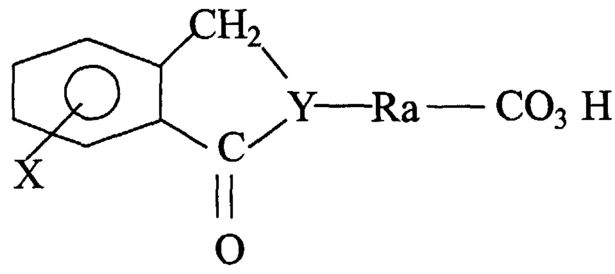

or

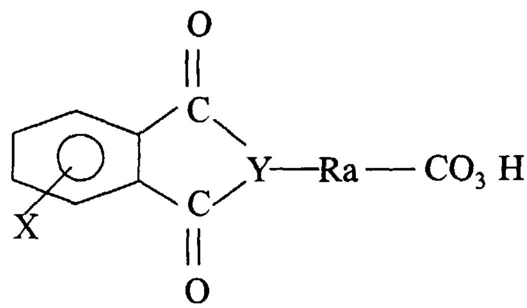

wherein Ra is a substituted or unsubstituted, saturated or unsaturated, linear or branched hydrocarbon group having from 1 to 14 carbon atoms, Y is an heteroatom and X are substituents in position ortho or meta independently selected from the group of hydrogen, hydroxy, halogen, carboxy, aliphatic saturated or unsaturated, linear or branched, hydrocarbon group having from 1 to 10 carbon atoms, or a mixture thereof.

Preferably Ra is a substituted or unsubstituted, linear or branched alkyl group or alkenyl group having from 2 to 12 carbon atoms, preferably from 2 to 10, more preferably from 2 to 8, even more preferably from 3 to 6 and most preferably 5 carbon atoms. Preferably Y is an heteroatom selected from the group consisting of oxygen, nitrogen and sulfur, and more preferably is nitrogen atom (>N-). Preferably X are substituents on position ortho or meta independently selected from the group consisting of hydrogen, hydroxy, aliphatic linear or branched alkyl group or alkenyl group having from 1 to 10 carbon atoms, preferably from 2 to 7 and most preferably from 3 to 5 carbon atoms. Highly preferred herein all the substituents X are independently hydrogen.

The preferred peroxyacids for use according to the present invention are pthalimido- and phtalamido peroxyalkanoic acids.

Highly preferred herein is ε-pthalimido peroxyhexanoic acid which may be commercially available from AUSIMONT under the name PAP ®, or EURECO ® (in granule form), Eureco WKC ® (in wet granule form) or Eureco HG ® (in powdered active form).

Other suitable oxidising agents for use herein include inorganic oxides like urea peroxide, potassium permanganate, ruthenium tetra-oxide, osmium tetra-oxide, cerium compounds including cerium oxides, cerium hydroxides, cerium hydrated oxides, cerium oxysalts and the like.

It has now surprisingly been found that the oxidising agents, preferably the peroxyacids and/or diacyl peroxides according to the present invention when used on top of the chelating agent as described herein provide significant improved odour control capacity versus odour associated with bodily fluids. More particularly their combination results in a synergistic odour reduction as compared to the odour reduction obtained for each of them used alone at the same total level in an absorbent article coming into contact with bodily fluids.

It is speculated that the oxidising agents herein prevent the generation of the odour by blocking the microbial and/or enzymatic activity. Indeed it is speculated that the oxidising agents herein, preferably peroxyacids and/or diacyl peroxides oxidize sensitive sulphidryl and sulphur bonds typically present in enzymes, thereby deactivating the enzymes which otherwise would have contributed to the normal metabolism of the micro-organisms like microbes. It is further speculated that these oxidising agents oxidize double bonds in other metabolites like for instance nutriments

(e.g., unsaturated fat) for the micro-organisms, thereby rendering these nutriments inefficient for the microbial growth which otherwise would have resulted in generation of malodorous compounds. It is further believed that these oxidising agents herein disrupt the chemiosmotic function of the lipoprotein cytoplasmatic membrane of the microbe/bacteria cells and thus disrupt the transport function at the cell walls. This later disruption is especially noticeable with the hydrophobic oxidising agents herein like the cyclic or cyclic alkyl peroxyacids namely those according to the chemical formulae described herein before and/or diacyl peroxides described herein before. By 'hydrophobic oxidising agent' it is meant those oxidising agents that can be solubilised in the lipidic layers of the cell wall of the micro-organisms. Highly preferred hydrophobic oxidising agents for use herein are the phthalimido- and phtalamido peroxyalkanoic acids, dibenzoyl peroxide, benzoyl lauroyl peroxide and/or dilauroyl peroxide.

Furthermore it has been found that the oxidising agents as described herein are able to combat the malodorous compounds already present by oxidising them into non-smelling compounds.

In a preferred embodiment of the present invention the oxidising agents are the peroxyacids (e.g., phtalimido peroxyalkanoic acid and/or phtalamido peroxyalkanoic acid) and/or the diacyl peroxides. Indeed in contrast to the use of some inorganic peroxides like persulfate or percarbonate, the peroxyacids and diacylperoxides as described herein are free of deactivation by catalase and/or peroxidase enzymes that are present in bodily fluids.

An additional advantage of the preferred oxidising agents herein like the peroxyacids and diacyl peroxides as described herein is that the generation of malodorous smelling by products like chlorine derivatives and ammonium derivatives is avoided, when they come into contact with bodily fluids.

Typically, the disposable absorbent articles according to the present invention comprise the oxidising agent or a mixture thereof at a level of 0.5 $g/m^2$ to 300 $g/m^2$ preferably from $3g/m^2$ to 250 $g/m^2$, more preferably from $6g/m^2$ to 150 $g/m^2$.

<u>Chelating agents</u>

The articles according to the present invention further comprise on top of the oxidising agent described herein before, at least one chelating agent or a mixture thereof.

Suitable chelating agents for use herein are those known to those skilled in the art. Suitable chelating agents include for examples phosphonate chelating agents, polyfunctionally-substituted aromatic chelating agents, amino carboxylate chelating agents, other chelating agents like ethylene diamine N,N'- disuccinic acid, aspartic acid, glutamic acid, malonic acid, glycine and mixtures thereof.

Suitable phosphonate chelating agents to be used herein may include ethydronic acid, alkali metal ethane 1-hydroxy diphosphonates as well as amino phosphonate compounds, including amino alkylene poly (alkylene phosphonate), alkali metal ethane 1-hydroxy diphosphonates, nitrilo trimethylene phosphonates, ethylene diamine tetra methylene phosphonates, aminotri(methylene phosphonates) (ATMP) and diethylene triamine penta methylene phosphonates. The phosphonate compounds may be present either in their acid form or as salts of different cations on some or all of their acid functionalities. Typically, these amino phosphonates do not contain alkyl or alkenyl groups with more than 6 carbon atoms. Preferred phosphonate chelating agents to be used herein are diethylene triamine penta methylene phosphonates (DETPMP). Such phosphonate chelating agents are commercially available from Monsanto under the trade name DEQUEST®.

Polyfunctionally-substituted aromatic chelating agents may also be useful in the compositions herein. See U.S. patent 3,812,044, issued May 21, 1974, to Connor et al. Preferred compounds of this type in acid form are dihydroxydisulfobenzenes such as 1,2-dihydroxy-3,5-disulfobenzene.

A preferred biodegradable chelating agent for use herein is ethylene diamine N,N'- disuccinic acid, or alkali metal, or alkaline earth, ammonium or substitutes ammonium salts thereof or mixtures thereof. Ethylenediamine N,N'-disuccinic acids, especially the (S,S) isomer have been extensively described in US patent 4, 704, 233, November 3, 1987. to Hartman and Perkins. Ethylenediamine N,N'-disuccinic acids is, for instance, commercially available under the tradename ssEDDS® from Palmer Research Laboratories.

Suitable amino carboxylate chelating agents to be used herein include ethylene diamine tetra acetates (EDTA), diethylene triamine pentaacetates, diethylene triamine pentaacetate (DTPA), N-hydroxyethylethylenediamine triacetates, nitrilotri-acetates, ethylenediamine tetrapropionates, triethylenetetraaminehexa-acetates, diethylenetriamine pentaacetates, ethanol-diglycines, propylene diamine tetracetic acid (PDTA) and methyl glycine di-acetic acid (MGDA), both in their acid form, or in their alkali metal, ammonium, and substituted ammonium salt forms. Particularly suitable amino carboxylates to be used herein are ethylene diamine tetra acetates (EDTA), diethylene triamine penta acetic acid (DTPA) or mixture thereof

The more preferred chelating agents for use herein are selected from ethylenediamine tetracetate, -triacetate, -diacetate, and -monoacetate, ethylenediamine N,N,-disuccinic acid (sodium salt), ethylenediamine penta (methylene phosphonic acid) (sodium salt), ethylenediamine tetra (methylene phosphonic acid) or mixtures thereof Most preferably the chelating agent is ethylene diamine tetracetate.

Typically, the disposable absorbent articles according to the present invention comprise the chelating agent or a mixture thereof at a level of 1 $g/m^2$ to200 $g/m^2$ preferably from 5 $g/m^2$ to 100 $g/m^2$, more preferably from 10 $g/m^2$ to 50 $g/m^2$.

The present invention is based on the finding that the oxidising agent herein, especially the peroxyacids and/or diacyl peroxides together with a chelating agent, especially ethylene diamine tetraacetates, deliver a synergistic odour control benefit especially when used in an absorbent article coming into contact with bodily fluids.

Indeed, it is speculated that the chelating agents not only prevent the formation of malodorous components that would otherwise have resulted from the degradation of components in presence of metal ions but also allow to retain the oxidising capacity of the oxidising agent over prolonged periods of time by reducing its degradation by metal ions when it comes into contact with for example bodily fluids.

<u>Optional agents</u>

The articles according to the present invention preferably further comprise other conventional agents or mixtures thereof.

For instance additional odour control agents or combinations thereof, known in the art for this purpose may be used herein. These agents can typically be classified according to the type of odour the agent is intended to combat. Odors may be chemically classified as being acidic, basic or neutral.

Alternatively, the odor control agents may be categorized with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carbonates (e.g., sodium carbonate), bicarbonates (e.g., sodium bicarbonate), phosphates (e.g., sodium phosphate), sulphates (e.g., zinc and copper sulphates), carboxylic acids such as citric acid, lauric acid, boric acid, adipic acid and maleic acid, activated carbons, clays, zeolites, silicas, cyclodextrine and derivatives thereof and starches. Such odor control agents and systems are disclosed in more details hereinafter and for example in EP-A- 348 978, EP-A- 510 619, WO 91/12029, WO 91/11977, WO 91/12030, WO 81/01643, US5429628 and WO 96/06589.

Another suitable odor control agent for use herein is a buffer system, such as citric acid and sodium bicarbonate, sodium phosphate and sorbic acid buffer systems. Also, buffer systems having a pH of from 7 to 10 as described for example in WO94/25077 may be useful herein.

Alternative odor control agents are ion exchange resins such as those described in US 4 289 513 and US 3340875.

Masking agents such as perfumes may also be used as odor control agents herein.

Typically, the disposable absorbent articles according to the present invention may comprise the additional odour control agent or a mixture thereof at a level of from 0.5 $gm^{-2}$ to 600 $gm^{-2}$, preferably from 5 to 500 $gm^{-2}$, more preferably from 10 $gm^{-2}$ to 350 $gm^{-2}$ and most preferably from 20 $gm^{-2}$ to 200 $gm^{-2}$

<u>Silica odor control agent</u>

Particularly suitable herein as an additional odor control agent is silica. Silica, i.e. silicon dioxide $SiO_2$ exists in a variety of crystalline forms and amorphous modifications, any of which are suitable for use herein. In particular, silicas having a high surface area or in agglomerated form are preferred. Silica molecular sieves are not considered to be within the definition of silica as used herein. Preferably the silica is in a highly purified form such that is contains at least 90%, preferably 95%, more preferably 99% silicon dioxide. Most preferably the silica is silica gel having a 100% silica content. Alternatively, the silica may be provided from other sources such as metal silicates including sodium silicate.

<u>Zeolite odor control agent</u>

The use and manufacture of zeolite material is well know in the literature and is described in the following reference texts: ZEOLITE SYNTHESIS, ACS Symposium Series 398, Eds. M. L. Occelli and H. E Robson (1989) pages 2-7; ZEOLITE MOLECULAR SIEVES, Structure, Chemistry and Use, by D. W. Breck, John Wiley and Sons (1974) pages 245-250, 313-314 and 348-352; MODERN APPLICATIONS OF MOLECULAR SIEVE ZEOLITES, Ph.D. Dissertation of S. M. Kuznicki, U. of Utah (1980), available from University of Microfilms International, Ann Arbor, Michigan, pages 2-8.

Zeolites are crystalline aluminosilicates of group IA and group IIA elements such as Na, K, Mn, Ca and are chemically represented by the empirical formula: $M_{2/n}O \cdot Al_2O_3 \cdot {}_ySiO_2 \cdot wH_2O$ where y is 2 or greater, n is the cation valence, and w is the water content in the voids of the zeolite.

Structurally, zeolites are complex, crystalline inorganic polymers based on an infinitely extending framework of AlO4 and SiO4 tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

$$M_{x/n} [(AlO_2)_x (SiO_2)_y] . wH_2O$$

where n is the valence of cation M, w is the number of water molecules per unit cell, x and y are the total number of tedrahedra per unit cell, y/x usually having values of 1-5.

Zeolites may be naturally derived or synthetically manufactured. The synthetic zeolites being preferred for use herein. Suitable zeolites for use herein include zeolite A, zeolite P, zeolite Y, zeolite X, zeolite DAY, zeolite ZSM-5, or mixtures thereof. Most preferred is zeolite A.

According to the present invention the zeolite is preferably hydrophobic. This is typically achieved by increasing the molar ratio of the SiO2 to AlO2 content such that the ratio of x to y is at least 1, preferably from 1 to 500, most preferably from 1 to 6.

<u>Carbon material</u>

The carbon material suitable for employment herein is the material well known in the art as an absorber for organic molecules and/or air purification purposes. Carbon suitable for use herein is available form a number of commercial sources under the trade names such as CALGON Type "CPG", Type SGL, Type "CAL" and type "OL". Often such material is referred to as "activated" carbon or "activated" charcoal. Typically it is available in the form of an extremely fine, dusty particles having large surface areas (200 - several thousand $m^2$/g.) It is to be understood that any of the "air purifying" or "activated" carbons of commerce can be used in the practice of this invention.

The articles according to the present invention may further comprise other conventional agents like absorbent gelling materials.

<u>Absorbent gelling materials</u>

As is well-known from recent commercial practice, absorbent gelling materials (sometimes referred to as "super-sorbers") are becoming broadly used in absorbent articles. AGM's are materials which have fluid-absorbing properties.

Such materials form hydrogels on contact with water (e.g., with urine, blood, and the like). One highly preferred type of hydrogel-forming, absorbent gelling material is based on polyacids, especially polyacrylic acid. Hydrogel-forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supematant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the absorbent article, which is undesirable from a consumer aesthetics standpoint. Furthermore, rate of fluid absorption can be affected by particle size. Larger particles have very much reduced rates of absorption. Preferred for use herein are absorbent gelling material s particles substantially all of which have a particle size of from about 30 microns to about 2mm. "Particle Size" as used herein means the weighted average of the smallest dimension of the individual particles.

Typically, the disposable absorbent articles according to the present invention may comprise an absorbent gelling material particle or mixtures thereof at levels ranging from $10gm^{-2}$ to 300 $gm^{-2}$, preferably from $30gm^{-2}$ to $150gm^{-2}$, more preferably from $55gm^{-2}$ to $85gm^{-2}$.

<u>The disposable absorbent article</u>

The odor control system (i.e., oxidizing agent as described herein and chelating agent as well as the additional odour control agent and/or absorbing gelling material if present) may be incorporated into the absorbent article by any of the methods disclosed in the art, for example layered on the core of the absorbent article or mixed within the fibres of the absorbent core.

The oxidizing agent as described herein and the chelating agent are preferably incorporated between two layers of cellulose tissue. Optionally the system may be bonded between two cellulose tissue layers with, for example, a hot melt adhesive or any suitable bonding system, as described in WO 94/01069.

In one embodiment of the present invention the oxidizing agent as described herein and the chelating agent are incorporated in a layered structure in accordance with the disclosure of WO 94/01069 or Italian patent application number TO 93A 001028. TO 93A 001028 describes a layered structure substantially as described in WO 94/01069 with the exception that TO 93A 001028 comprises a much higher quantity of absorbent gelling material in the intermediate layer which is between the fibrous layers ($120gm^{-2}$) that would be incorporated as an optional component in the present invention. The intermediate layer comprises in particular a polyethylene powder as thermoplastic material which is

mixed with the chelating agent and oxidizing agent. The mixture is then heated such that the polyethylene melts and glues the laminate layers. Adhesive lines are preferably also placed on the edges of the laminate to ensure that the edges of the laminate stick and any loose oxidising agent as described herein and chelating agent present do not fall out of the laminate.

Alternatively, the polyethylene powder may be replaced by a conventional glue for instance those commercially available from ATO Findley under the name H20-31® to glue the laminate layers and/or components together. Advantageously this method step allows to avoid the heating step necessary when using polyethylene powder.

The oxidizing agent and the chelating agent may be distributed homogeneously or non homogeneously over the entire absorbent article or in at least one layer of the topsheet or in at least one layer of the core or any mixture thereof. The oxidizing agent and the chelating agent may be distributed homogeneously or non homogeneously on the whole surface of the desired layer or layers, or on one or several area of the surface layer/layers to which it is positioned (e.g. central area and/or surrounding area like the edges of a layer of the absorbent article) or mixtures thereof. Preferably the oxidizing agent is located towards the topsheet or is located in the topsheet itself (preferably the secondary topsheet).

In a preferred embodiement the oxidizing agent is positioned such that at least a portion of the fluid discharge comes into contact with said oxidizing agent and chelating agent before the optional additional odour control agent (e.g., silica/zeolite) and/or AGM if present. In particular, the oxidizing agent and chelating agent are located in a separate layer from the optional additional odour control agent and/or AGM if present. Preferably the oxidizing agent and the chelating agent arelocated towards the topsheet or are located in the topsheet itself (preferably the secondary topsheet) and the optional additional odour control agent and/or AGM if present are located further away from the topsheet than the oxidizing agent. In one embodiment of the present invention, the oxidizing agent and the chelating agent are positioned in at least one of the topsheet layers and the optional additional odour control agent and/or AGM if present are positioned in the core. More preferably, the oxidizing agent and the chelating agent are located at the fluid discharge entry point of the absorbent article.

The oxidizing agent as described herein, the chelating agent and optional additional odour control agent may be independently incorporated as a powder or a granulate or can be sprayed in the form of an oxidising agent-containing solution within the absorbent article. When used in a granulate or particulate form the oxidizing agent, the chelating agent and the optional odour control agent and AGM if present may be granulated separately and then mixed together or granulated together.

Typical disposable absorbent articles according to the preferred embodiments of the present invention are those as described herein after:

<u>Absorbent core</u>

According to the present invention, the absorbent can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent may have any thickness depending on the end use envisioned.

a <u>Primary/Secondary Fluid Distribution Layer</u>

One optional component of the absorbent 'according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers. In particular fibrous layers maintain the capillaries between fibers even when wet are useful as distribution layers.

b <u>Fluid Storage Layer</u>

Positioned in fluid communication with, and typically underlying the primary or secondary distribution layers, is a fluid storage layer. The fluid storage layer can comprise any usual absorbent material or combinations thereof It preferably comprises absorbent gelling materials in combination with suitable carriers.

Suitable carriers include materials which are conventionally utilized in absorbent structures such as natural,

modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

c <u>Optional Fibrous ("Dusting") Layer</u>

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

d <u>Other Optional Components of the absorbent structure</u>

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

<u>The topsheet</u>

According to the present invention the absorbent article comprises as an essential component a topsheet. The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as woven and non woven fabrics and films.

In a preferred embodiment of the present invention at least one of the layers, preferably the upper layer, of the topsheet comprises a hydrophobic, liquid permeable apertured polymeric film. Preferably, the upper layer is provided by a film material having apertures which are provided to facilitate liquid transport from the wearer facing surface towards the absorbent structure. If present the lower layer preferably comprises a non woven layer, an apertured formed film or an airlaid tissue.

The term apertured polymeric topsheet as used herein refers to topsheets comprising at least one layer or a multiplicity of layers wherein at least one layer is formed from a continuous or uninterrupted film material wherein apertures are created. It has been surprisingly discovered that apertured polymeric film topsheets yield significantly better odour control than other types of topsheets such as for example thermal bonded nonwoven materials.

In general the apertured polymeric topsheet of the present invention is compliant, soft feeling, and non-irritating to the wearer's skin. Further, the topsheet is fluid pervious permitting fluids (e.g., menses and/or urine) to readily penetrate through its thickness. Suitable apertured polymeric film topsheets for use herein include polymeric apertured formed films, thermoplastic films, apertured plastic films, and hydroformed thermoplastic films; porous foams; net-like

reticulated foams and thermoplastic films; and thermoplastic scrims.

Preferred topsheets for use in the present invention are selected from apertured formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Each of these patents are incorporated herein by reference. Particularly preferred microapertured formed film topsheets are disclosed in U.S. patent 4,609,518 (Curro et al), issue September 2, 1986 and U.S. patent 4,629,643 (Curro et al), issued December 16, 1986, which are incorporated by reference. The preferred topsheet for the present invention is the formed film described in one or more of the above patents and marketed on sanitary napkins by The Procter & Gamble Company of Cincinnati, Ohio as "DRI-WEAVE."

Suitable topsheets in the field of three dimensional formed film are described in EP 0 018 020 and EP 0 059 506. Especially preferred in a three dimensional formed polymeric topsheet having openings in the shape of regular pentagons which are regularly spaced and have an opening of 0.41 square millimeter. The openings are spaced 0.37 square millimeters apart transversely and 0.25 millimeters longitudinally. This topsheet has an initial opening (preforming ) thickness of about 25m a final (post forming) thickness of about 0.53mm and an open area of from 25% to about 40%.

Another formed film topsheet which is especially preferred is one having openings of two shapes; regular pentagons having an area of about 0.21 square millimeters and an irregular hexagon having an area of 1.78 square millimeters. The openings are distributed so that the distance between the sides of the figures is about 0.37 mm to about 0.42mm. The preforming and post forming film thickness are respectively 0.25 and 0.43 mm. This film has an open area of about 33.7%. Both films are made according to the teachings of the above mentioned patents.

A third form suitable topsheet comprises two separate perforated polymeric films superimposed one on the other.

The body surface of the formed film topsheet of the present invention may also be hydrophilic so as to help liquid to transfer through the topsheet faster than if the body surface was not hydrophilic. In this manner the likelihood that menstrual fluid will flow off the topsheet rather than flowing into and being absorbed by the absorbent structure is diminished. In a preferred embodiment, surfactant is incorporated into the polymeric materials of the formed film topsheet such as is described in U.S. Patent Application Serial No. 07/794,745, "Absorbent Article Having A Nonwoven and Apertured Film Coversheet" filed on November 19, 1991 by Aziz, et al., which is incorporated by reference. Alternatively, the body surface of the topsheet can be made hydrophilic by treating it with a surfactant such as is described in the above referenced U.S. 4,950,254, incorporated herein by reference.

<u>The backsheet</u>

The backsheet primarily prevents the extrudes absorbed and contained in the absorbent structure from welling articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film typically having a thickness of from about 0.012 mm (0.5 mil) to about 0.051 mm (2.0 mil).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or mall finished to provide a more clothlike appearance. Further, the backsheet can permit vapors to escape from the absorbent structure, i.e. be breathable, while still preventing extrudates from passing through the backsheet. Also breathable backsheets comprising several layers, e.g. film plus non-woven structures, can be used.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867 US 3 156 242 and European Patent Application number 95120653.1.

Suitable dual or multi layer breathable backsheets for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

Particularly preferred are backsheets meeting the requirements as defined in European Patent Application number 96830343.8 and more preferably wherein the absorbent article also meets the requirements as described therein.

According to the present invention the breathable backsheet comprises at least one, preferably at least two water vapour permeable layers. Suitable water vapour permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2 dimensional planar layers of the backsheet may be made of any material known in the art, but are preferably manufactured from commonly available polymeric materials. Suitable materials are for example GORE-TEX (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 637 819 and US 4 591 523.

Suitable macroscopically expanded films for use herein include films as described in for example US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours International S.A., Switzerland.

According to the present invention the backsheet may comprise in addition to said water vapour permeable layer additional backsheet layers. Said additional layers may be located on either side of said water vapour permeable layer of the backsheet. The additional layers may be of any material, such as fibrous layers or additional water vapour permeable layers as described herein above.

<u>Odor control test</u>

The odor reduction is measured by for example an in vitro sniff test. This test consists in analyzing the products (including references) by expert graders that express their judgment about (un)pleasantness of the odor of the

product (pad).

The present invention is further illustrated by the following examples.

Examples:

Example A

The feminine pads used in the following examples were Always (Always is a registered Trade Mark) as sold by the Procter & Gamble Company.

Each feminine pad was opened by cutting the wrap around the perforated coverstock at its bottom face approximately along a longitudinal edge of the release paper which covers the external adhesive layer. The side of the absorbent fibrous core was then exposed by slightly shifting the water impermeable plastic bottom layer and subsequently, the fibrous core was split into two halves, each having approximately the same thickness, along a plane which is parallel to the plane of the napkin itself. The oxidising agent and the chelating agent were homogeneously mixed and homogeneously distributed between these two fibrous layers which were then joined together to reconstitute the absorbent core.

The water impermeable inner backsheet was then put back into its original position and the wrap around perforated coverstock was sealed along the cut by means of e.g. a double sided adhesive tape.

Samples were produced using the method above, containing the odor control systems as described hereinbelow.

The oxidising agent (0.3g) used was ε-phtalimido peroxyhexanoic acid commercially available from Ausimont or dibenzoyl peroxide commercially available from AKZO NOBEL under the name Lucidol® or benzoyl lauroyl peroxide. The chelating agent used was (0.1g) of ethylenediamine tetracetate (EDTA) available from BASF AG.

Example B

In Example B samples were produced using the same method as in Example A, except that AGM (0.8g) available from Dow Chemicals (XZ 9589001), and silica (1.0g) Syloblanc 82 available from Grace GmbH or zeolite (0.8g) Zeolite A, Wessalith CS, available from Degussa AG were added to the oxidising agent and chelating agent.

Example C

Other pads were prepared by following the method in Example A above except that after having split the fibrous core into two halves, the oxidising agent and the chelating agent were homogeneously distributed onto the upper halve fibrous layer (i.e. the fibrous layer halve intended to be closer to the topsheet) and AGM was homogeneously distributed onto the lower halve fibrous layer (i.e., the one intended to be closer to the backsheet of the pad once reconstituted). Then a layer of airlaid tissue (19 mm* 70 mm of low basis weight) (available from Fripa under the code/name NCB Tissue HWS) was positioned between the two halve fibrous layers which are then joined together to reconstitute the absorbent core. The presence of the airlaid tissue between the two fibrous layer avoids direct contact between the oxidizing agent and AGM.

These samples were produced using as the oxidising agent (0.3 g) of either ε-phtalimido peroxyhexanoic acid commercially available from Ausimont or dibenzoyl peroxide commercially available from AKZO NOBEL under the name Lucidol® or benzoyl lauroyl peroxide. The AGM (0.8g) used was available from Dow Chemicals (XZ 9589001). The chelating agent used was 0.1 g of ethylenediamine tetracetate available from BASF AG.

Example D

In Example D samples were produced using the same method as in Example C, except that silica (1.0g) Syloblanc 82 available from Grace GmbH and/or zeolite (0.8g) Zeolite A, Wessalith CS, available from Degussa AG were added on top of AGM onto the lower halve fibrous layer

All these pads were found to provide outstanding odour control benefits when in contact with bodily fluids. Especially it was observed that there was a synergistic odour reduction when combining the oxidising agent like the peroxyacid together with the chelating agent like ethylenediamine tetracetate as compared to the use of each of them alone at the same total level in the absorbent article coming into contact with bodily fluids.

**Claims**

1. A disposable absorbent article comprising as an odour control system for controlling odour, preferably odour associated with bodily fluids, at least one oxidizing agent together with at least one chelating agent.

**2.** An article according to claim 1 wherein said article is a sanitary napkin, pantiliner, tampon, diaper, incontinent pad, breast pad, perspiration pad or interlabial pad.

**3.** An article according to any of the preceding claims wherein said article comprises a liquid pervious topsheet, a backsheet and an absorbent core intermediate said backsheet and said topsheet.

**4.** An article according to claim 3 wherein at least one of the layers, preferably the upper layer, of the topsheet comprises an apertured polymeric film.

**5.** An article according to any of the preceding claims wherein said oxidizing agent is selected from the group consisting of peroxygen bleaches, inorganic oxides, cerium compounds and mixture thereof, preferably is hydrogen peroxide, a percarbonate, a persilicate, a persulphate, a perphosphate, a peroxyacid, an alkyl hydroperoxide, a peroxide, a diacyl peroxide, an ozonide, a supereoxide, an oxo-ozonide, a periodate, a salt thereof or a mixture thereof and more preferably is a peroxyacid and/or a diacyl peroxide.

**6.** An article according to any of the preceding claims wherein said oxidizing agent is a peroxyacid according to the following formula:

$$R_3\text{-CO3H}$$

wherein $R_3$ is a substituted or unsubstituted, saturated or unsaturated, linear or branched hydrocarbon group having from 1 to 25 carbon atoms or a cyclic group having from 3 to 32 carbon atoms and optionally at least one heteroatom, or a cyclic alkyl group having from 4 to 32 carbon atoms and optionally at least one heteroatom, or a mixture thereof

**7.** An article according to claim 6 wherein $R_3$ in the peroxyacid formula is a substituted or unsubstituted, linear or branched, alkyl group or alkenyl group having from 1 to 25 carbon atoms, or an aryl alkyl group having from 4 to 32 total carbon atoms, or an aryl group having from 3 to 32 carbon atoms, or an heterocyclic group having from 3 to 32 carbon atoms and from 1 to 5 hetero atoms, or an heterocyclic alkyl group containing from 4 to 32 total carbon atoms and from 1 to 5 hetero atoms, wherein the hetero atoms are independently selected from the group consisting of oxygen, nitrogen and sulfur, and preferably is nitrogen or oxygen.

**8.** An article according to any of the preceding claims wherein the oxidising agent is a peroxyacid according to the formulae:

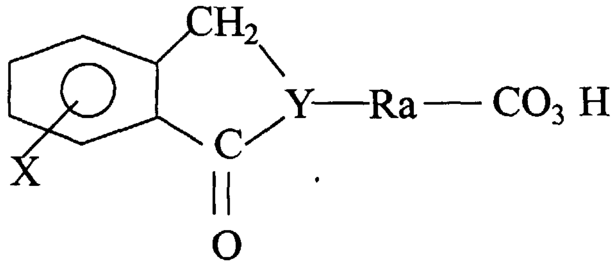

or

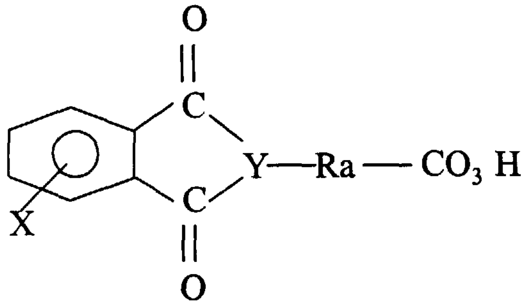

wherein Ra is a substituted or unsubstituted, saturated or unsaturated, linear or branched hydrocarbon group having from 1 to 14 carbon atoms, Y is an heteroatom, preferably selected from the group consisting of oxygen, nitrogen and sulfur and more preferably is nitrogen, and X are substituents in position ortho or meta independently selected from the group of hydrogen, hydroxy, halogen, carboxy, aliphatic saturated or unsaturated, linear or branched, hydrocarbon group having from 1 to 10 carbon atoms, or a mixture thereof.

**9.** An article according to any of the preceding claims wherein said oxidizing agent is a diacyl peroxide according to the formula:

$$R_1\text{-C(O)-O-O-(O)C-}R_2$$

wherein $R_1$ and $R_2$ are the same or different and are selected from the group of substituted or unsubstituted, saturated or unsaturated, linear, branched or cyclic hydrocarbon groups having from 1 to 50 carbon atoms, preferably from 2 to 40 and more preferably from 4 to 30 carbon atoms, or a mixture thereof.

**10.** An article according to claim 9 wherein $R_1$ and $R_2$ in the diacyl peroxide formula are independently a mono or polycyclic aromatic ring, a homo or heteroaromatic ring, substituted or unsubstituted of from 2 to 50 total carbon atoms, preferably a benzene, or an aliphatic group having from 2 to 40 carbon atoms and wherein the diacyl peroxide is most preferably a benzoyl alkanoyl peroxide wherein the alkanoyl group has from 4 to 20 carbon atoms or a mixture thereof

**11.** An article according to any of the preceding claims wherein the oxidizing agent is a pthalimido peroxyalkanoic acid, a phtalamido peroxyalkanoic acid, dilauroyl peroxide, dibenzoyl peroxide and/or benzoyl lauroyl peroxide.

**12.** An article according to any of the preceding claims which comprises from 0.5 g/$m^2$ to 300 g/$m^2$ preferably from 3g/$m^2$ to 250 g/$m^2$, more preferably from 6g/$m^2$ to 150 g/$m^2$ of said oxidizing agent or a mixture thereof.

**13.** An article according to any of the preceding claims wherein the chelating agent is selected from the group consisting of phosphonate chelating agents, amino phosphonate chelating agents, polyfunctionally-substituted aromatic

chelating agents, amino carboxylate chelating agents, other chelating agents like ethylene diamine N,N'- disuccinic acid, aspartic acid, glutamic acid, malonic acid, glycine and mixtures thereof.

**14.** An article according to any of the preceding claims wherein said chelating agent is selected from the group consisting of ethylenediamine tetracetate, -triacetate, -diacetate, and -monoacetate, ethylenediamine N,N,-disuccinic acid, ethylenediamine penta (methylene phosphonic acid), ethylenediamine tetra (methylene phosphonic acid) or mixtures thereof and more preferably is ethylene diamine tetracetate.

**15.** An article according to any of the preceding claims which comprises from 1 $g/m^2$ to 200 $g/m^2$ preferably from 5 $g/m^2$ to 100 $g/m^2$, more preferably from 10 $g/m^2$ to 50 $g/m^2$ of a chelating agent or mixture thereof.

**16.** An article according to any of the preceding claims, which further comprises an additional odour control agent typically selected from the group consisting of silicas, zeolites, carbons, starches, cyclodextrine, pH buffered materials, chitin, kieselguhr, clays, ion exchange resins, carbonates, bicarbonates, phosphates, sulphates, carboxylic acids and combination thereof and preferably is a silicate, a zeolite or a combination thereof.

**17.** An article according to claim 16 which comprises from 0.5 $gm^{-2}$ to 600 $gm^{-2}$, preferably from 5 to 500 $gm^{-2}$, more preferably from 10 $gm^{-2}$ to 350 $gm^{-2}$ and most preferably from 20 $gm^{-2}$ to 200 $gm^{-2}$ of the odour control agent or a mixture thereof.

**18.** An article according to any of the preceding claims which further comprises at least one absorbent gelling material.

**19.** An article according to claim 18, which comprises the absorbent gelling material or a mixture thereof at a level of $10gm^{-2}$ to $300gm^{-2}$, preferably from $30gm^{-2}$ to $150gm^{-2}$, more preferably from $55gm^{-2}$ to $85gm^{-2}$.

**20.** The use, as an odour control system, of an oxidising agent, preferably a peroxyacid and/or a diacyl peroxide, together with a chelating agent, preferably ethylenediamine tetra-acetate.

European Patent Office

# EUROPEAN SEARCH REPORT

Application Number
EP 99 10 3909

## DOCUMENTS CONSIDERED TO BE RELEVANT

| Category | Citation of document with indication, where appropriate, of relevant passages | Relevant to claim | CLASSIFICATION OF THE APPLICATION |
|---|---|---|---|
| X | WO 95 22655 A (WEYERHAEUSER CO) 24 August 1995 (1995-08-24)<br>* tables 1,2 *<br>* figures 8,9 *<br>* page 26, line 41 - line 43 *<br>* page 32, line 8 - line 12 *<br>* page 41, line 16 - line 17 *<br>--- | 1-3,5, 9-20 | A61L15/46 |
| D,X | US 4 363 322 A (ANDERSSON BROR A E) 14 December 1982 (1982-12-14)<br>* column 2, line 13 - line 66 *<br>* column 3, line 49 - line 53 *<br>* column 4, line 11 *<br>* column 4, line 21 - line 34 *<br>* column 12, line 1 - line 2 *<br>--- | 1-3,5, 9-13,16, 20 | |
| X | EP 0 604 919 A (ISHIHARA SANGYO KAISHA) 6 July 1994 (1994-07-06)<br>* page 2, line 48 - line 50 *<br>* page 3, line 8 - line 10 *<br>* page 4, line 37 - line 44 *<br>* page 7, line 16 - line 21 *<br>* claims 1-3,9 *<br>--- | 1,2, 16-19 | TECHNICAL FIELDS SEARCHED<br>A61L<br>A61F |
| D,X<br><br>Y | EP 0 510 619 A (KIMBERLY CLARK CO) 28 October 1992 (1992-10-28)<br>* page 5, line 5 *<br>* page 8, line 14 - line 34 *<br>* claims 1,19,20 *<br>--- | 1-5, 12-19<br>1,5-8 | |
| Y | WO 92 11238 A (INTEROX CHEMICALS LTD) 9 July 1992 (1992-07-09)<br>* page 2, line 19 - page 3, line 16 *<br>* page 12, line 15 - page 13, line 31 *<br>* page 19, line 20 - line 25 *<br>---<br>-/-- | 1,5-8 | |

The present search report has been drawn up for all claims

| Place of search | Date of completion of the search | Examiner |
|---|---|---|
| THE HAGUE | 6 August 1999 | Thornton, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 99 10 3909

## DOCUMENTS CONSIDERED TO BE RELEVANT

| Category | Citation of document with indication, where appropriate, of relevant passages | Relevant to claim | CLASSIFICATION OF THE APPLICATION |
|---|---|---|---|
| D,A | EP 0 811 391 A (PROCTER & GAMBLE) 10 December 1997 (1997-12-10) * see claims * ----- | 1-3, 13-17,20 | |
| | | | TECHNICAL FIELDS SEARCHED |

The present search report has been drawn up for all claims

| Place of search | Date of completion of the search | Examiner |
|---|---|---|
| THE HAGUE | 6 August 1999 | Thornton, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 10 3909

This annex lists the patent family members relating to the patent documents cited in the above-mentioned European search report.
The members are as contained in the European Patent Office EDP file on
The European Patent Office is in no way liable for these particulars which are merely given for the purpose of information.

06-08-1999

| Patent document cited in search report | Publication date | Patent family member(s) | Publication date |
|---|---|---|---|
| WO 9522655 A | 24-08-1995 | US 5547541 A | 20-08-1996 |
| | | AU 1921395 A | 04-09-1995 |
| | | CA 2181476 A | 24-08-1995 |
| | | EP 0745160 A | 04-12-1996 |
| | | JP 9509226 T | 16-09-1997 |
| | | US 5807364 A | 15-09-1998 |
| US 4363322 A | 14-12-1982 | SE 423311 B | 03-05-1982 |
| | | DE 2914386 A | 25-10-1979 |
| | | GB 2023430 A | 03-01-1980 |
| | | SE 7804195 A | 14-10-1979 |
| | | SE 7809038 A | 29-02-1980 |
| EP 0604919 A | 06-07-1994 | JP 6254389 A | 13-09-1994 |
| EP 0510619 A | 28-10-1992 | AU 1501192 A | 29-10-1992 |
| | | CA 2054095 A | 23-10-1992 |
| | | JP 5123358 A | 21-05-1993 |
| | | MX 9201838 A | 01-08-1993 |
| WO 9211238 A | 09-07-1992 | CA 2099009 A | 23-06-1992 |
| | | EP 0563186 A | 06-10-1993 |
| | | JP 6504047 T | 12-05-1994 |
| | | US 5466825 A | 14-11-1995 |
| EP 0811391 A | 10-12-1997 | AU 3147197 A | 05-01-1998 |
| | | AU 3370197 A | 05-01-1998 |
| | | CA 2257541 A | 11-12-1997 |
| | | CA 2257628 A | 11-12-1997 |
| | | EP 0811392 A | 10-12-1997 |
| | | WO 9746190 A | 11-12-1997 |
| | | WO 9746194 A | 11-12-1997 |

EPO FORM P0459

For more details about this annex : see Official Journal of the European Patent Office, No. 12/82